# EUROPEAN PATENT APPLICATION

(11) **EP 4 506 690 A1**
(43) Date of publication of application: **12.02.2025**
(21) Application number: 23778514.2
(22) Date of filing: 31.03.2023
(51) Int. Cl.: G01N 33/48, G01N 1/28, G01N 1/38

(54) **DISPOSABLE SPECIMEN COLLECTION BOX**

(30) Priority: 02.04.2022 CN 202210343457
(71) Applicant: Zhuhai Keyu Biological Engineering Co., Ltd., Zhuhai, Guangdong 519040 (CN)
(72) Inventor: LV, Shengqiu, Zhuhai, Guangdong 519040 (CN); SUN, Liping, Zhuhai, Guangdong 519040 (CN); HUANG, Yanchang, Zhuhai, Guangdong 519040 (CN)
(74) Representative: Michalski Hüttermann & Partner Patentanwälte mbB
(86) International application number: PCT/CN2023/085739
(87) International publication number: WO 2023/186150

(57) **Abstract**

A disposable specimen collection box, comprising a bottle body (1), a sealing cover (2), and a collection spoon (21), wherein the interior of the bottle body (1) is divided into a mixing cavity (11) and an egg concentration chamber (12) in communication with the mixing chamber (11) via a filter screen (130); and the collection spoon (21) is eccentrically and rotatably arranged on an inner side of the sealing cover (2). A docking portion (212), which is arranged at an upper portion of the collection spoon (21), and a docking member (40) are respectively loaded via an upper and a lower opening of a second sleeve (30), and the docking portion (212) and the docking member (40) are fixedly connected to each other in the second sleeve (30); and then the second sleeve (30) is fixedly inserted into the first sleeve (209), such that the upper portion of the collection spoon (21) can be assembled on the first sleeve (209) more conveniently and can rotate relatively to the first sleeve (209) and the second sleeve (30). A stop edge (41) of the docking member (40) is not required to deform during assembly, and can thus be machined to have sufficient strength. Therefore, the docking member (40) cannot be separated from the second sleeve downwards. In addition, the egg concentration efficiency and effect are improved by means of the arrangement manner of cavities inside the bottle body (1) and the arrangement manner of the filter screen (130).

## Description

### TECHNICAL FIELD

The present invention belongs to the technical field of medical devices, and specifically relates to a disposable specimen collection box used for stool collection and analysis.

### BACKGROUND

Disposable specimen collection box is one of the commonly used medical consumables for sample analysis, such as: cooperating with the stool analyzer to complete the examination and analysis of the patient's stool. Current disposable specimen collection boxes generally include a bottle body and a sealing cover. The interior of the bottle body is divided into a mixing chamber and an egg collection chamber, and at least some portions of the mixing chamber and the egg collection chamber are communicated through a filter screen. A collection spoon is eccentrically arranged on the sealing cover. The collection spoon is inserted into the mixing chamber after collecting the sample. The collection spoon is rotatable. After the collection spoon rotates for mixing, relevant detection is performed.

However, there are still some aspects that the prior art can be further improved, such as:
The patent document with publication number CN111207946 A discloses a disposable specimen collection cup and a puncture mixing method therefor. A drive head is integrally formed in the upper end of the collection spoon, and a flange is provided on the top of the drive head. The flange is provided with a rotary needle fitting groove for matching a rotary needle (a device configured on the testing equipment and used for driving the collection spoon to rotate), and a small section of external threads are provided on the side wall under the flange. A sleeve (the "positioning base" recorded in patent document CN111207946 A) is eccentrically provided on the inner side of the sealing cover. During assembly, the drive head is rigidly (squeezing the flange inward) installed into the sleeve from the lower end of the cylindrical, and meanwhile, the external threads of the drive head and a small section of internal threads on the inner wall of the sleeve are in screw-joint fit. At this time, the flange of the drive head extends a certain distance from the upper end of the sleeve. During use, the rotary needle drives the collection spoon to rotate, and the external threads of the drive head and the internal threads of the sleeve are tripped first. At this time, the flange of the drive head is supported on the upper end of the sleeve.

In the above prior art, the flange is required to have a certain degree of elasticity during assembly so as to be installed into the cylindrical chamber and extend out of the upper end of the sleeve. However, the flange is required to have high strength during use so as to be reliably supported on the upper end of the sleeve and prevent the drive head from being detached from the sleeve due to the downward force of the rotary needle. To conclude from the above text, the above structure adopted in the prior art is difficult to achieve optimal results in both assembly and usage. In addition, the setting methods of the chambers and filter screen are relatively simple, and the egg collection efficiency and effect also need to be further improved in the prior art.

### SUMMARY

The present invention aims to provide a disposable specimen collection box with a more stable structure and more convenient assembly. The present invention is implemented through the following technical solutions.

The present invention provides a disposable specimen collection box, including a bottle body, a sealing cover, and a collection spoon, wherein an interior of the bottle body is divided into a mixing chamber and an egg collection chamber in communication with the mixing chamber through a filter screen; wherein the collection spoon is eccentrically and rotatably arranged on an inner side of the sealing cover, and inserted into the mixing chamber when the sealing cover is fitted to the bottle body; wherein a cover body of the sealing cover is provided with a collection spoon drive port at a position corresponding to the collection spoon, wherein,
the disposable specimen collection box also includes a first sleeve fixedly arranged on the inner side of the sealing cover at a position corresponding to the collection spoon drive port, a second sleeve is inserted into an opening at a lower end of the first sleeve and fixedly connected to the first sleeve, and a docking member is inserted into an opening at an upper end of the second sleeve and matched rotatably with the second sleeve; wherein a stop edge is arranged on an outer periphery of an upper end of the docking member for supporting at the upper end of the second sleeve, and a docking portion is arranged on an upper portion of the collection spoon and is fixedly connected to the docking member in the second sleeve; and wherein a rotary needle fitting hole is provided in the center of the docking member and/or the docking portion.

Further, a lower end of the docking member is higher than a lower end of the second sleeve, an inner wall of the lower end of the second sleeve is provided with a section of internal threads, and the collection spoon is provided with a section of external threads below the docking portion for screw-joint fit with the internal threads.

Further, the second sleeve and the first sleeve are fixedly connected through buckles and slots provided on both of their sleeve wall in touch.

Further, the docking member is a third sleeve, and the docking portion is inserted into an opening at a lower end of the third sleeve and is fixedly connected to the third sleeve; the docking portion and the third sleeve are fixedly connected through buckles and slots provided between them.

Further, the cover body of the sealing cover is provided with a liquid injection port and a collection port at a position corresponding to the egg collection chamber.

Further, an exterior side of the cover body of the sealing cover is covered with a puncturable membrane.

Further, the interior of the bottle body is divided into a vertical mixing chamber and a vertical egg collection chamber by a vertical partition wall.

Further, the interior of the bottle body is divided into a vertical mixing chamber and an L-shaped egg collection chamber by an L-shaped partition wall, the L-shaped egg collection chamber includes a vertical chamber and a transverse chamber, the lower end of the vertical chamber is communicated with one end of the transverse chamber, and the mixing chamber is located above the transverse chamber; the L-shaped partition wall includes a vertical partition wall and a transverse partition wall, and the vertical partition wall and the transverse partition wall are corresponding mounted with a first filter screen and a second filter screen, through which the mixing chamber and the egg collection chamber are communicated with each other.

Further, the first filter screen includes at least one filter screen, of which the upper end is located at the 2/3 to 3/4 position from bottom to top of the mixing chamber, and of which the lower end is close to the bottom of the mixing chamber, and of which the plane connecting the left and right ends passes through or is close to the axis of the mixing chamber.

Further, the first filter screen includes three filter screens, the vertical partition wall is an arc partition wall and is provided with three filter windows arranged side by side, and the three filter screens are installed in the three filter windows respectively.

Further, the transverse partition wall is inclined downward from the vertical partition wall, and the bottom of the transverse chamber is inclined toward the vertical chamber.

Further, an egg collection tank is provided at a connection between the vertical chamber and the transverse chamber, and the lowest part of the bottom of the transverse chamber is connected to the egg collection tank.

Further, the vertical chamber of the egg collection chamber has an egg collection guiding slope inside, which extends from bottom toward top and passes through the first filter screen, and the lower end of the egg collection guiding slope is connected to the egg collection tank.

Further, the upper end of the mixing chamber is provided with a collection spoon insertion port, and an internal side wall of the collection spoon insertion port is provided with a quantitative collection ring, which is an annular structure convexly extending toward the axial direction of the collection spoon insertion port.

Further, an outer surface of the bottle body is provided with a testing shooting surface, which is located at the outer side of the mixing chamber and faces the spoon portion of the collection spoon.

Compared with the prior art, the beneficial effects of the present invention are:
1. According to the present invention, the docking portion provided on the upper portion of the collection spoon and the docking member are respectively installed from the upper and lower openings of the second sleeve, the docking portion and the docking member are fixedly connected to each other in the second sleeve, and then the second sleeve is fixedly sleeved in the first sleeve, so that the upper portion of the collection spoon can be more conveniently assembled on the first sleeve, and can rotate relatively to the first sleeve and the second sleeve. In the present invention, the stop edge of the docking member is not required to deform during assembly, and can thus be machined to have a sufficient strength. When the rotary needle of the testing equipment is matched with the rotary needle fitting hole, its force will not cause the docking member and the docking portion to be detached downward from the second sleeve. Moreover, the rotary needle fitting hole can be made very small, and the corresponding rotary needle can also be small, making it easier for the rotary needle to pierce the puncturable membrane.
2. In one mode of the present invention, a mixing chamber and an L-shaped egg collection chamber are installed in the bottle body, the mixing chamber is located above the transverse chamber of the egg collection chamber, and meanwhile, a first filter screen and a second filter screen are respectively arranged on the vertical partition wall and the transverse partition wall. On one hand, it is possible to allow the specimen in the mixing chamber to enter the egg collection chamber from both the first filter screen and the second filter screen at the same time, greatly improving the egg collection efficiency and effect; on the other hand, it is ensured that the specimen at the bottom of the mixing chamber can smoothly and efficiently enter the transverse chamber through the second filter screen, avoiding the defective problem that the specimen precipitation of the existing specimen collection box accumulates at the bottom of the mixing chamber, and further improving detection and making the analysis results more accurate.
3. In one mode of the present invention, by using the first filter screen, of which the upper end is located at the 2/3 to 3/4 position from bottom to top of the mixing chamber, the lower end is close to the bottom of the mixing chamber, and the plane connecting the left and right ends passes through or is close to the axis of the mixing chamber, the efficiency and quantity of the mixed specimens entering the egg collection chamber is further improved, thereby improving the egg collection efficiency and effect, improving detection, and thus improving the accuracy of analysis results.
4. In one mode of the present invention, in order to improve the egg collection effect of specimens and improve detection, the bottom surface of the transverse chamber is set to tilt toward the vertical chamber, and the lowest part of the bottom surface of the transverse chamber is connected to the egg collection tank at the connection between the vertical chamber and the transverse chamber. Moreover, the vertical chamber of the egg collection chamber is internally provided with an egg collection guiding slope connected to the egg collection tank.

### BRIEF DESCRIPTION OF DRAWINGS

FIG. 1 is a schematic diagram of an assembled disposable specimen collection box according to Embodiment 1 of the present invention.
FIG. 2 is a schematic diagram showing the separation of the sealing cover and collection spoon from the bottle body in the disposable specimen collection box according to Embodiment 1 of the present invention.
FIG. 3 is an exploded schematic diagram of the disposable specimen collection box according to Embodiment 1 of the present invention.
FIG. 4 is a top view of the disposable specimen collection box according to Embodiment 1 of the present invention.
FIG. 5 is a sectional view in the A-A direction in FIG. 4 of the disposable specimen collection box according to Embodiment 1 of the present invention.
FIG. 6 is a cross-sectional view in the B-B direction in FIG. 5 of the disposable specimen collection box according to Embodiment 1 of the present invention.
FIG. 7 is a axial-sectional view of another implementation of chamber partitioning in the disposable specimen collection box according to Embodiment 2 of the present invention.

### DETAILED DESCRIPTION

In order to make the technical solution of the present invention clearer and the technical advantages more clear, the technical solution of the present invention will be clearly and completely described below in conjunction with specific embodiments. Obviously, the described embodiments are merely some of the embodiments of the present invention, not all of them, and the technical features of the embodiments in the present invention can be combined with each other on the premise that they do not conflict with each other.

Specific implementations of the present invention will be further described below in conjunction with the accompanying drawings. For ease of explanation, orientations are defined in the present invention in conjunction with the accompanying drawings. The definitions of these orientations are merely for the purpose of clearly describing relative positional relationships and are not used to limit the actual orientation of products or apparatuses during production, use, sales, etc. In addition, the terms "first" and "second" are merely used for descriptive purposes and cannot be understood as indicating or implying relative importance or implicitly indicating the quantity of indicated technical features. Furthermore, in the embodiments of the present invention, unless otherwise clearly stated and limited, terms such as "installation", "setting", "connection", and "fixing" should be understood in a broad sense, which, for example, may be a fixed connection, a detachable connection, or integrally formed; may be a direct connection or an indirect connection through an intermediary. For those of ordinary skill in the art, the specific meanings of the above terms in the present invention can be understood according to specific circumstances.

The specific implementations of the present invention will be further described below in conjunction with the accompanying drawings.

### Embodiment 1

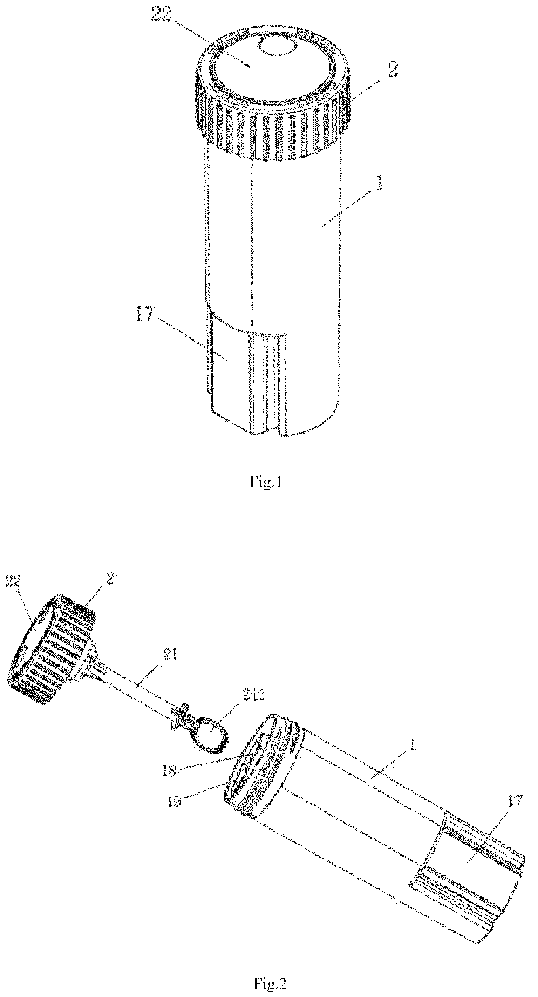

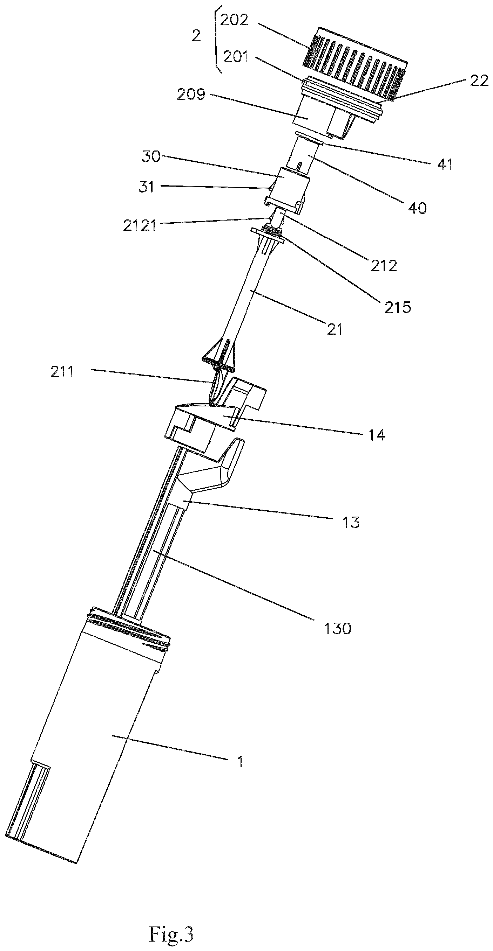

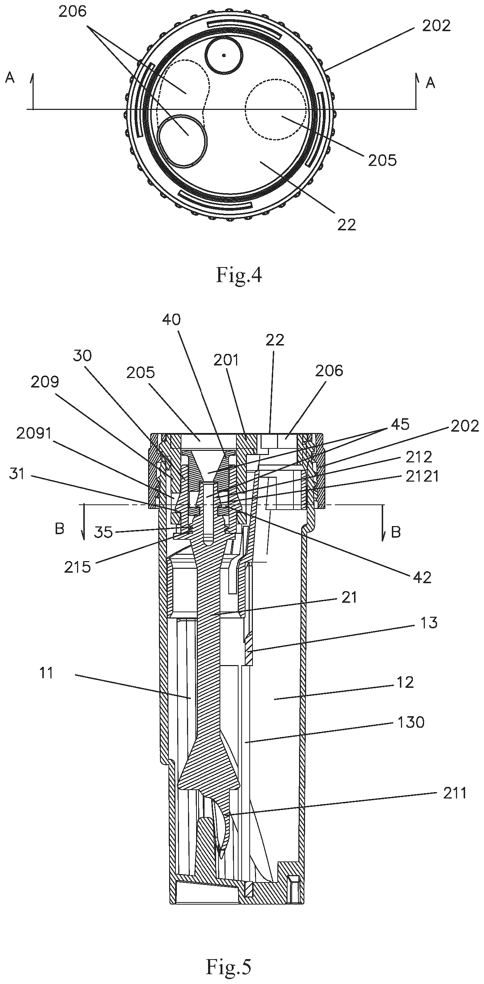

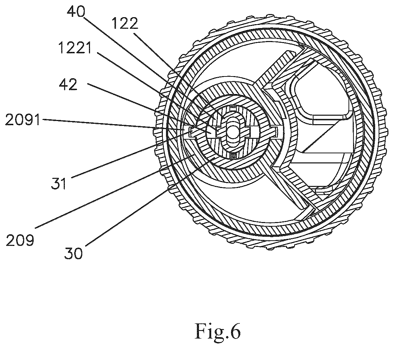

Referring to FIGS. 1 to 6, a disposable specimen collection box according to this embodiment includes a bottle body 1 and a sealing cover 2. The sealing cover 2 includes a cover body 201 and a screw joint portion 202 provided on an outer periphery of the cover body 201. The sealing cover 2 is detachably assembled on an opening of an upper end of the bottle body 1 in a sealing manner. The interior of the bottle body 1 is divided into a vertical mixing chamber 11 and a vertical egg collection chamber 12 by a vertical partition wall 13. The mixing chamber 11 is communicated with the egg collection chamber 12 through a filter screen 130 provided on the partition wall 13. A collection spoon 21 is eccentrically and rotatably arranged on an inner side of the sealing cover 2. The collection spoon 21 is inserted into the mixing chamber 11 when the sealing cover 2 is fitted to the bottle body 1.

The cover body 201 of the sealing cover 2 is provided with a collection spoon drive port 205 at a position corresponding to the collection spoon 21 for, after the collection spoon 21 collects a specimen and injects liquid, a rotary needle of a testing equipment to be inserted and drive the collection spoon 21 to rotate for specimen mixing. A liquid injection and collection port 206 is defined at a position corresponding to the egg collection chamber 12 and is used for injecting liquid into the bottle body 1 after the collection spoon 21 collects specimen, or collecting specimen liquid externally from the inside of the bottle 1 after the specimen is mixed.

An outer side of the cover body 201 is covered with a puncturable membrane 22, which is an elastic soft film that can automatically shrink to a sealing state after the puncturable membrane is pierced and the needle is withdrawn. The puncturable membrane 22 covers the collection spoon drive port 205 and the liquid injection and collection port 206 in a sealing manner. A first sleeve 209 is fixedly arranged on the inner side of the sealing cover 21 at a position corresponding to the collection spoon drive port 205. The first sleeve 209 can be fixedly connected to or integrally formed on the inner side of the cover body 201.

The disposable specimen collection box according to this embodiment also includes a second sleeve 30 and a docking member 40. The second sleeve 30 is inserted into an opening at a lower end of the first sleeve 209 and is fixedly connected to the first sleeve 209. The docking member 40 is inserted into an opening at an upper end of the second sleeve 30 and matched with the second sleeve 30 in a rotating manner (that is, the docking member 40 can rotate within the second sleeve 30). A stop edge 41 is arranged on an outer periphery of an upper end of the docking member 40, which can support on the upper end of the second sleeve and prevent the docking member 40 from detaching downward relative to the second sleeve 30. The collection spoon 21 is provided with a spoon portion 211 at a lower portion thereof and a docking portion 212 at an upper portion thereof. The docking portion 212 is fixedly connected to the docking member 40 in the second sleeve 30 and rotates with the docking member40 relative to the second sleeve 30. A rotary needle fitting hole 45 is provided in a center of the docking member 40 and/or docking portion 212 for docking with the rotary needle of the testing equipment. Upon being driven by the rotary needle, the docking member 40 and the docking portion 212 will rotate relatively to the second sleeve 30.

Specifically, the docking member 40 is a third sleeve, and the docking portion 212 of the collection spoon 21 is inserted into an opening at a lower end of the third sleeve and is fixedly connected to the third sleeve. In addition, an outer wall of the second sleeve 30 is provided with a buckle 31, and a sleeve wall of the first sleeve 209 is provided with a slot 2091. The buckle 31 and the slot 2091 cooperate with each other for a fixed connection between the second sleeve 30 and the first sleeve 209, limiting rotational and axial movements therebetween. Similarly, an outer wall of the docking portion 212 is provided with a buckle 2121, and a barrel wall of the third sleeve is provided with a slot 42. The buckle 2121 and the slot 42 cooperate with each other for a fixed connection between the docking portion 212 and the docking member 40, limiting rotational and axial movements therebetween.

In this embodiment, a lower end of the docking member 40 is higher than a lower end of the second sleeve 30. An inner wall of the lower end of the second sleeve 30 is provided with a section of internal threads 35. The collection spoon 21 is provided with a section of external threads 215 below the docking portion 212 for meshing with the section of internal threads 35.

When assembling the disposable specimen collection box according to the above embodiment, the docking portion 212 and the docking member 40 are respectively installed from upper and lower openings of the second sleeve 30 and are fixedly connected to each other in the second sleeve, and then the second sleeve is fixedly sleeved in the first sleeve, so that the upper portion of the collection spoon 21 can be more conveniently assembled on the first sleeve, and can rotate relative to the first sleeve and the second sleeve. In this embodiment, the stop edge 41 of the docking member 40 is not required to deform during assembly, and can thus be machined to have sufficient strength. When the rotary needle of the testing equipment is matched with the rotary needle fitting hole, its acting force will not cause the docking member 40 and the docking portion 212 to be detached downward from the second sleeve. Moreover, the rotary needle fitting hole can be made very small, and the corresponding rotary needle can also be small, making it easier for the rotary needle to pierce the puncturable membrane. In addition, during assembly, the external threads 215 on an upper portion of the collection spoon 21 are in screw-joint fit with the internal threads 35 on the inner wall of the lower end of the second sleeve 30. At this time, the stop edge 41 of the docking member 40 extends beyond the upper end of the second sleeve 30 for a certain distance. During use, the rotating needle drives the docking member 40 and the docking portion 212 to rotate, so that the internal threads 35 and the external threads 215 are tripped first. At this time, the stop edge 41 of the docking member 40 is supported on the upper end of the second sleeve 30, and the collection spoon 21 rotates in the mixing chamber 11 and mixes the specimen carried on it into the injected diluent.

### Embodiment 2

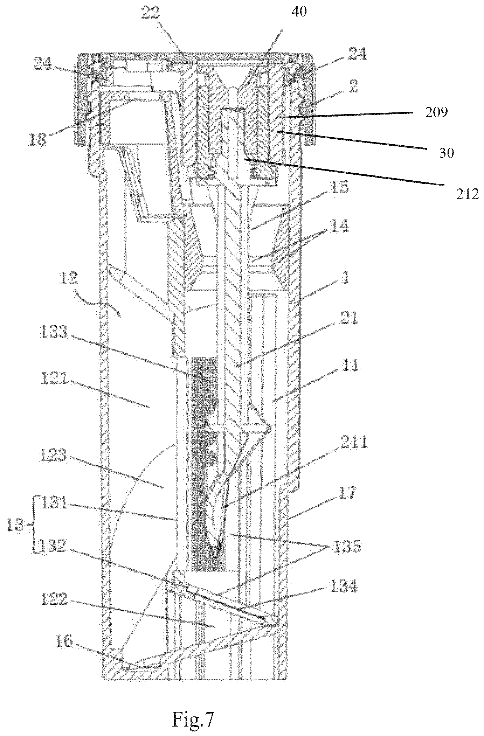

As shown in FIG. 7, the difference between Embodiment 2 and Embodiment 1 lies in that: In the second embodiment, the mixing chamber 11 is a vertical chamber, and the egg collection chamber 12 is an L-shaped chamber. The egg collection chamber 12 includes a vertical chamber 121 and a transverse chamber 122. A lower end of the vertical chamber 121 is communicated with one end of the transverse chamber 122, and the mixing chamber 11 is located above the transverse chamber 122. Correspondingly, the partition wall 13 is L-shaped and includes a vertical partition wall 131 and a transverse partition wall 132 (the angle between the vertical partition wall 131 and the transverse partition wall 132 is preferably 110-145 degrees). The vertical partition wall 131 and the transverse partition wall 132 are corresponding respectively provided with a first filter screen 133 and a second filter screen 134 through which the mixing chamber 11 is communicated with the egg collection chamber 12.

When the disposable specimen collection box of the present invention is placed on a test tube rack of a stool analyzer for testing, the stool analyzer first adds diluent to the mixing chamber 11, and then drives the collection spoon 21 to rotate and stirs to mix the specimen and the diluent thoroughly and evenly. Finally, the specimen enters into the vertical chamber 121 and the transverse chamber 122 of the egg collection chamber 12 through the first filter screen 133 and the second filter screen 134 respectively, and is collected into the egg collection tank 16.

In this embodiment, on one hand, it is possible to allow the specimen in the mixing chamber 11 to enter the egg collection chamber 12 from both the first filter screen 133 and the second filter screen 134 at the same time, greatly improving the egg collection efficiency and effect; on the other hand, it is ensured that the specimen at the bottom of the mixing chamber 11 can smoothly and efficiently enter the transverse chamber 122 through the second filter screen 134, avoiding a defective problem that the specimen in the existing specimen collection box deposits and accumulates at the bottom of the mixing chamber 11, and further improving detection and making the analysis results more accurately.

In one possible implementation, in order to further improve the efficiency and quantity of the mixed specimen entering the egg collection chamber 12, thereby improving the egg collection efficiency and effect, improving detection, and thus improving the accuracy of analysis results, the first filter screen 133 includes at least one filter screen, of which the upper end is located at the 2/3 to 3/4 position from bottom to top of the mixing chamber 11, and of which the lower end is close to the bottom of the mixing chamber 11, and of which a plane connecting the left and right ends passes through or is close to the axis of the mixing chamber 11.

Specifically, the first filter screen 135 includes three filter screens. The vertical partition wall 131 is an arc partition wall and is provided with three filter windows 135 arranged side by side. The three filter screens are installed in the three filter windows 135 respectively.

The bottom of the transverse chamber 122 is inclined toward the vertical chamber 121. The egg collection tank 16 is provided at a connection between the vertical chamber 121 and the transverse chamber 122. The lowest part of the bottom surface of the transverse chamber 122 is connected to the egg collection tank 16 to further improve the egg collection effect of specimens, thereby improving detection.

As further shown in FIG. 7, the vertical chamber 121 of the egg collection chamber 12 has an egg collection guiding slope 123 inside, which extends from bottom toward top and passes through the first filter screen 133. A lower end of the egg collection guiding slope 123 is connected to the egg collection tank 16. The egg collection guiding slope 123 can guide the specimen that enters into the vertical chamber 121 through the first filter screen 133 to the egg collection tank 16, which on the one hand improves the egg collection speed and on the other hand improves the egg collection effect.

As shown in FIG. 7, the upper end of the mixing chamber 11 is provided with a collection spoon insertion port 15. An internal side wall of the collection spoon insertion port 15 is provided with a quantitative collection ring 14, which is an annular structure convexly extending toward the axial direction of the collection spoon insertion port 15. Specifically, a surface of the quantitative collection ring 14 is an arc-shaped smooth surface, the upper and lower sides of which make a smoothly transition to the internal side wall of the collection spoon insertion port 15. After using the spoon portion 211 of the collection spoon 21 to take samples, the collection spoon 21 is inserted into the mixing chamber 11 through the collection spoon insertion port 15. In this process, the quantitative collection ring 14 will block and scrape off the excess specimen from the spoon portion 211 of the collection spoon 21 to achieve a purpose of quantitative sampling, making the sampling volume meet the standards of the stool analyzer, ensuring that the stool analyzer can fully dilute and mix the taken specimen by adding diluent into the mixing chamber 11, effectively improving the mixing efficiency and effect, thereby ensuring the accuracy of analysis results.

As shown in FIGS. 1, 2 and 7, the upper surface of the sealing cover 2 is provided with a puncturable membrane 22, which is an elastic soft film that can automatically shrink to a sealing state after the puncturable membrane is pierced and the needle is withdrawn, and covers a mixing rotating shaft 23 connected to the upper end of the collection spoon 21 on the sealing cover 2, as well as a liquid adding hole 18 communicated with the mixing chamber 11 and a collection hole 19 communicated with the egg collection chamber 12 at the upper end of the bottle 1 in a sealing manner. The sealing cover 2 is a hard cover body, which is connected to the upper end of the bottle body 1 through a threaded structure. A sealing member 24 is provided at the connection with the upper end of the bottle body 1. The puncturable membrane 22 is a silicone membrane, and the sealing member 24 is a silicone sealing ring. During piercing and mixing, a liquid adding needle of the stool analyzer pierces the puncturable membrane 22 and is pulled out after the diluent is added, the puncturable membrane 22 automatically shrinks to the sealing state, and then a mixing needle of the stool analyzer pierces the puncturable membrane 22 again and is matched with the drive head at the upper end of the collection spoon 21 to drive the collection spoon 21 to rotate and stir in the mixing chamber 11. The disposable specimen collection box has good sealing throughout the entire process, ensuring the safety, reliability, hygiene and pollution-free testing and analysis.

As shown in FIGS. 1-3, an outer surface of the bottle body 1 is provided with a testing shooting surface 17, which is located at the outer side of the mixing chamber 11 and faces the spoon portion 211 of the collection spoon 21 (i.e., the testing shooting surface 17 faces a position where the spoon portion 211 collects the specimen), so that a directional shooting can be achieved.

The above implementations are merely preferred implementations of the present invention and cannot be used to limit the scope of protection of the present invention. Any non-substantive changes and substitutions made by those skilled in the art on the basis of the present invention fall within the scope of the claimed protection of the present invention.

## Claims

1. A disposable specimen collection box, comprising a bottle body, a sealing cover, and a collection spoon, wherein an interior of the bottle body is divided into a mixing chamber and an egg collection chamber in communication with the mixing chamber through a filter screen; wherein the collection spoon is eccentrically and rotatably arranged on an inner side of the sealing cover, and inserted into the mixing chamber when the sealing cover is fitted to the bottle body; wherein a cover body of the sealing cover is provided with a collection spoon drive port at a position corresponding to the collection spoon, wherein
the disposable specimen collection box further comprises a first sleeve fixedly arranged on the inner side of the sealing cover at a position corresponding to the collection spoon drive port, a second sleeve is inserted into an opening at a lower end of the first sleeve and fixedly connected to the first sleeve, and a docking member is inserted into an opening at an upper end of the second sleeve and matched rotatably with the second sleeve; wherein a stop edge is arranged on an outer periphery of an upper end of the docking member for supporting at the upper end of the second sleeve, and a docking portion is arranged on an upper portion of the collection spoon and is fixedly connected to the docking member in the second sleeve; and wherein a rotary needle fitting hole is provided in the center of the docking member and/or the docking portion.

2. The disposable specimen collection box according to claim 1, wherein a lower end of the docking member is higher than a lower end of the second sleeve, and wherein an inner wall of the lower end of the second sleeve is provided with a section of internal threads, and the collection spoon is provided with a section of external threads below the docking portion for meshing with the section of internal threads.

3. The disposable specimen collection box according to claim 2, wherein the second sleeve and the first sleeve are fixedly connected through buckles and slots provided on both of their sleeve wall in touch.

4. The disposable specimen collection box according to claim 2, wherein the docking member is a third sleeve, and the docking portion is inserted into an opening at a lower end of the third sleeve and is fixedly connected to the third sleeve; and wherein the docking portion and the third sleeve are fixedly connected through buckles and slots provided between them.

5. The disposable specimen collection box according to claim 1, wherein the cover body of the sealing cover is provided with a liquid injection port and a collection port at a position corresponding to the egg collection chamber.

6. The disposable specimen collection box according to claim 1, wherein an outer side of the cover body of the sealing cover is covered with a puncturable membrane.

7. The disposable specimen collection box according to claim 1, wherein the interior of the bottle body is divided into a vertical mixing chamber and a vertical egg collection chamber by a vertical partition wall.

8. The disposable specimen collection box according to claim 1, wherein the interior of the bottle body is divided into a vertical mixing chamber and an L-shaped egg collection chamber by an L-shaped partition wall, wherein the L-shaped egg collection chamber comprises a vertical chamber and a transverse chamber, a lower end of the vertical chamber being communicated with one end of the transverse chamber, and the vertical mixing chamber being located above the transverse chamber; and wherein the L-shaped partition wall comprises a vertical partition wall and a transverse partition wall corresponding respectively mounted with a first filter screen and a second filter screen, through which the vertical mixing chamber and the L-shaped egg collection chamber are communicated with each other.

9. The disposable specimen collection box according to claim 8, wherein the transverse partition wall is inclined downward from the vertical partition wall, and the bottom of the transverse chamber is inclined toward the vertical chamber.

10. The disposable specimen collection box according to claim 9, wherein an egg collection tank is provided at a connection between the vertical chamber and the transverse chamber, and the lowest part of the bottom of the transverse chamber is connected to the egg collection tank.
